# EUROPEAN PATENT SPECIFICATION

(11) **EP 2 855 358 B1**
(45) Date of publication and mention of the grant of the patent: **18.10.2017**
(21) Application number: 13747865.7
(22) Date of filing: 03.06.2013
(51) Int. Cl.: C01G 39/06

(54) **SULFUR-MOLYBDENUM CLUSTER AND METHOD FOR MANUFACTURING THE SAME**
SCHWEFEL-MOLYBDÄN-CLUSTER UND VERFAHREN ZUR HERSTELLUNG DAVON
AGRÉGAT DE SOUFRE-MOLYBDÈNE ET SON PROCÉDÉ DE FABRICATION

(30) Priority: 01.06.2012 US 201261654482 P
(43) Date of publication of application: 08.04.2015
(73) Proprietor: Université de Rennes 1, 35065 Rennes Cedex (FR); Université de Lorraine, 54052 Nancy Cedex (FR); Triskem International, 35170 Bruz (FR); Centre National de la Recherche Scientifique (C.N.R.S.), 75794 Paris Cedex 16 (FR)
(72) Inventor: BOUQUET, Valérie, F-35700 Rennes (FR); GUILLOUX-VIRY, Maryline, F-35131 Pont-Péan (FR); POTEL, Michel, F-35700 Rennes (FR); BOULANGER, Clotilde, F-57420 Coin les Cuvry (FR); LECUIRE, Jean-Marie, F-57420 Coin les Cuvry (FR); BOURSICOT, Stéphane, F-35190 Tinténiac (FR); BOMBARD, Aude, F-35170 Bruz (FR)
(74) Representative: Osha Liang
(86) International application number: PCT/IB2013/001470
(87) International publication number: WO 2013/179138

(56) References cited:
- WO-A1-2012/010761
- S. BOURSICOT; V. BOUQUET; 1. PERON; T. GUIZOUAM; M. POTEL; M. GUILLOUX-VIRY: "Synthesis of CuMoSpowders and thin films from intermediate oxides prepared by polymeric precursor method", SOLID STATE SCIENCES, vol. 14, 18 March 2012 (2012-03-18), pages 719-724, XP0028484531, ISSN: 1293-2558, DOI: 10.1016/J.SOLIDSTATESCIENCES.2012.03.013 cited in the application

## Description

### TECHNICAL FIELD OF THE INVENTION

The present disclosure relates to sulfur-molybdenum cluster compositions and their manufacturing process. The present disclosure relates particularly to sulfur-molybdenum cluster compositions of general formula MₓMo₆S₈ suitable for electrochemical applications, for example, for cation electrochemical insertion, detection, and analysis. The sulfur-molybdenum cluster compositions of the present disclosure may be for example deposited as thin films and used to measure the quantities of heavy metals present in various media such as polluted effluents.

### BACKGROUND

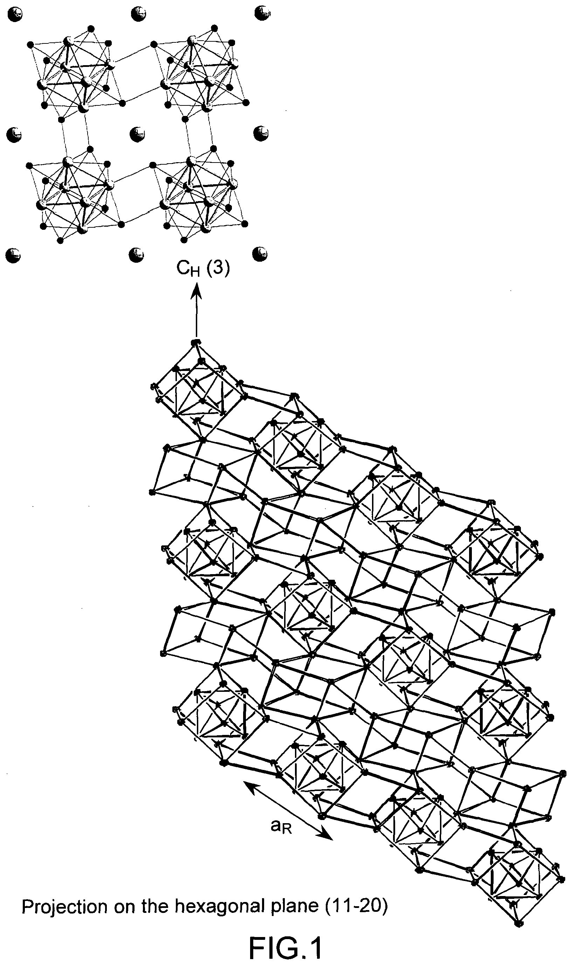

Since their discovery at the University of Rennes, the Chevrel phases (CP) have attracted many researches due to their very rich and varied properties. These compounds of general formula MₓMo₆X₈ where M is a cation and X is a chalcogen (S, Se or Te) present a 3D arrangement of Mo₆X₈ clusters which creates pseudo-cubic cavities that can receive cations (Figure 1). According to the nature, the size and oxidation degree of the cations, the CP present different electronic and magnetic properties. In addition, the 3D channel structure associated with high ionic mobility is highly favorable for insertion/de-insertion reactions. The electrochemical insertion of different cations such as heavy metals (*e.g.* Cd²⁺, Zn²⁺, Cu²⁺, Ni²⁺, Pb²⁺, Mn²⁺, Cr³⁺ and the like) has been thus demonstrated on CP bulk material and thin films. Based on these properties, manufacturing processes of Cu₂MoₙS₍ₙ₊₂₎ thin films have been recently published (WO 2009/007598, WO 2012/010761). The properties of insertion/de-insertion of these compounds may provide many application areas such as sensors of heavy metal ions in solution.

Indeed, the monitoring of the water pollution by heavy metals has become a major issue because the industrial activity generates many effluents containing metal cations whose impact on the environment can sometimes be particularly harmful. In order to respect the environmental standards, new sensors are required with specific criteria. For example, the sensors must have a high sensitivity (*e.g.* detection of traces elements), be easy to use and be miniaturized in order to provide compact analysis system (*e.g.* providing access to on-site analyses). The selectivity, the response time, the reversibility (re-use) and the reproducibility of the sensors are also determining factors.

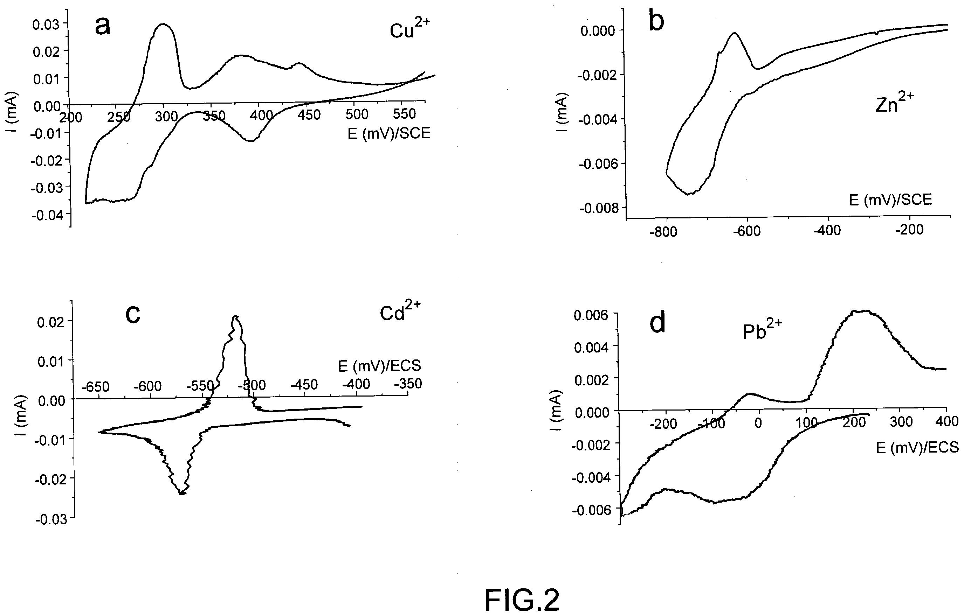

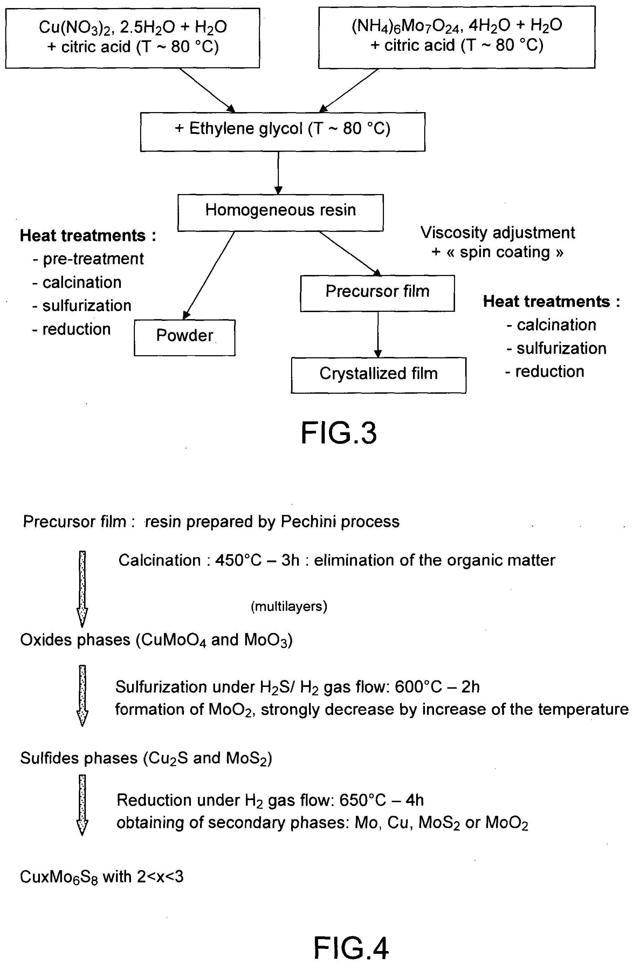

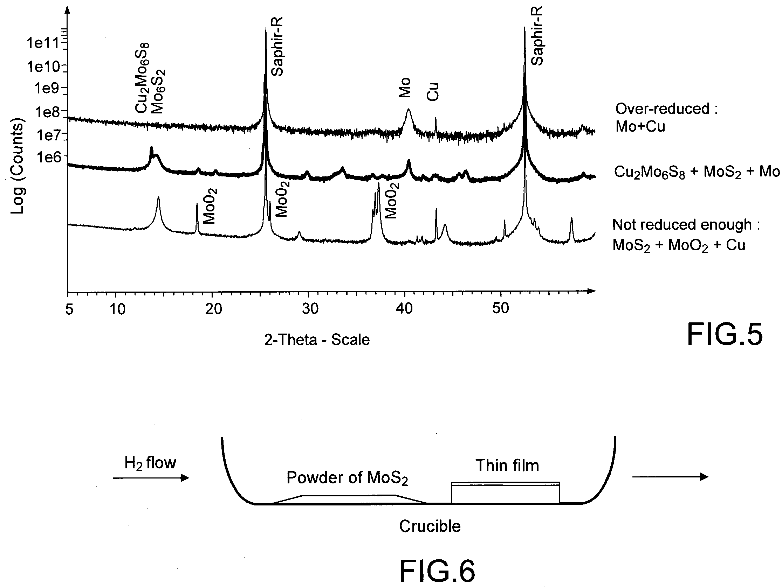

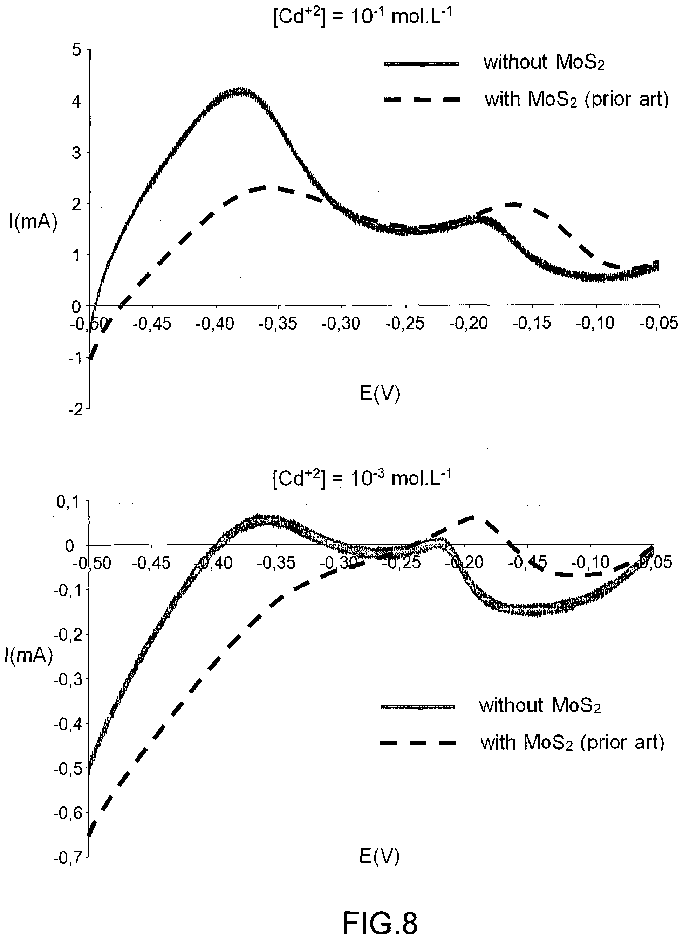

In this context, the insertion properties of MₓMo₆S₈ host networks by electrochemical route may be used to detect and measure pollutant ions. For this purpose, these MₓMo₆S₈ clusters may be synthesized in thin film form. The electrochemical insertion/de-insertion properties of Cu₂Mo₆S₈ (CMS) thin films prepared by pulsed laser deposition were successfully demonstrated [Z. Kaidi, C. Boulanger, J.M. Lecuire, N. Lemée, M. Guilloux-Viry, A. Perrin, Solid State Science, 1, 623 1999] as shown in Figure 2 (Temperature = 25°C, scan rate v = 60 mV.mn-1. a: 1 M CuSO₄ medium, b: 1 M ZnSO₄ medium, c:1 M CdSO₄ medium, d : 1 M PbNO₃ medium) but this synthesis method is not suitable for industrial process. CMS powders and thin films were synthesized by a chemical solution route [S. Boursicot, V. Bouquet, I. Péron, T. Guizouarn, M. Potel, M. Guilloux-Viry, Solid State Sciences 14, 2012, 719-724] (Figure 3) where a polymeric precursor resin was first prepared by the Pechini Process. The precursor thin films were deposited on sapphire by spin coating after viscosity adjustment of the polymeric precursor resin. They were then submitted to a 3 step heat treatment cycle: a calcination (to eliminate the organic matter and to synthesize the intermediate oxides), a sulfurization under 9 mol % H₂S/H₂ gas flow (to obtain a mixture of sulfides) and a reduction under H₂ gas flow. The optimization of the 3 step heat treatment cycle (Figure 4) led to the CMS phase but was usually not reproducible (Figure 5) due to the very small amount of matter used (some micrograms in case of thin films) and the difficulty to control the reduction gas flow suitable to treat such very small amount of matter. Moreover, the presence of secondary phases such as MoS₂, MoO₂ and Mo were generally observed, especially for very thin films, and depending on the applications, the presence of such secondary phases is prohibitive. For example, the electrochemical behavior is strongly affected by the presence of MoS₂ secondary phase (see prior art examples in Figure 8 referring to the presence of MoS₂) which leads to limited analytical determination capacities.

Accordingly, there exists a continuing need for providing sulfur-molybdenum cluster thin films suitable for electrochemical applications and having high analytical determination capacities, for example, for cation electrochemical insertion, detection, and/or analysis.

Further, there exists a continuing need for suitable and inexpensive industrial manufacturing processes of sulfur-molybdenum clusters thin films.

There exists also a continuing need for reproducible manufacturing processes of said sulfur-molybdenum clusters. For example, it is so far impossible to obtain reproducible compositions of very thin films, *i.e.,* when a precursor is deposited typically less than 5 times on a substrate.

### SUMMARY

A first aspect of the present disclosure is to provide a process for the preparation of a sulfur-molybdenum cluster composition, for example, in the form of a film such as a thin. The process comprises: preparing a precursor comprising a first molybdenum compound, calcinating the precursor, sulfurizing the precursor, and reducing the precursor in presence of a second molybdenum compound and of a reducing agent.

A further aspect of the present disclosure is to provide a film of sulfur-molybdenum cluster composition obtainable by the above-mentioned process.

A further aspect of the present disclosure is to provide an electrochemical apparatus comprising at least one of the above-mentioned film of sulfur-molybdenum cluster composition.

A further aspect of the present disclosure is to provide a method for detecting and/or quantifying the presence of at least one metal cation in a sample based on the insertion/de-insertion reaction of the at least one metal cation on at least one of above-mentioned film of sulfur-molybdenum cluster composition.

A further aspect of the present disclosure is to provide an electrode comprising at least one of the above-mentioned film of sulfur-molybdenum cluster composition.

A further aspect of the present disclosure is to provide a chalcogenide containing film based integrated device comprising at least one of the above-mentioned film of sulfur-molybdenum cluster composition. In some embodiments said device may be used as electrode, for example for photovoltaic applications.

### BRIEF DESCRIPTION OF DRAWINGS

Figure 1 shows the structure of Chevrel phases such as MₓMo₆X₈
Figure 2 shows cyclic voltammograms illustrating insertion/de-insertion reactions in Cu₂Mo₆S₈ thin films prepared by pulsed laser deposition.
Figure 3 shows the synthesis of Cu₂Mo₆S₈ by wet chemical route.
Figure 4 shows the heat treatment of thin films according to the prior art.
Figure 5 shows X Ray Diffraction (XRD) patterns of thin films according to the prior art.
Figure 6 shows a schematic view of the reduction step of an embodiment of the process of the present disclosure.
Figure 7 shows XRD patterns of sulfur-molybdenum cluster compositions of the present disclosure.
Figure 8 shows cyclic voltammograms illustrating the improvement of the electrochemical behavior of sulfur-molybdenum cluster compositions of the present disclosure compared to the prior art.
Figure 9 shows XRD patterns of thin films of sulfur-molybdenum cluster composition according to the present disclosure.
Figure 10 shows the electrochemical behavior of thin film of sulfur-molybdenum cluster composition of the present disclosure deposited on TiN/Si substrate.

### DETAILED DESCRIPTION

An objective of the present disclosure is to improve the reproducibility of the manufacturing process of sulfur-molybdenum cluster compositions, for example in the form of a film or a thin film, such as, for example, MₓMo₆S₈ thin films manufactured by a wet chemistry method such as chemical solution deposition. Another objective of the present disclosure is to decrease the amount of secondary phases such as MoS₂, MoO₂ and Mo secondary phases, and preferably to eliminate the MoS₂ secondary phase, from sulfur-molybdenum cluster compositions. Another objective of the present disclosure is to provide a wet chemical route for the synthesis of sulfur-molybdenum cluster compositions, for example in the form of thin films (for example, thickness ranging from about 80 nm to about 5 µm) or very thin films (for example, thickness ranging from about 20 nm to about 1 µm), preferably substantially free of MoS₂ secondary phase. Such thin film of sulfur-molybdenum cluster composition may be used, for example, as an electrode or by an electrochemical sensor for detecting and/or quantifying the presence of a metal cation in a sample. These and other objectives are attained by a process for the preparation of a film comprising a sulfur-molybdenum cluster composition, the process comprising: preparing a precursor comprising a first molybdenum compound, calcinating the precursor, sulfurizing the precursor, and reducing the precursor in presence of a second molybdenum compound and of a reducing agent.

In the present description and in the following claims, a composition substantially free from MoS₂ secondary phase is intended to indicate a composition comprising less than about 5 mol % of MoS₂ secondary phase (x-ray diffraction detection threshold), preferably less than about 2 mol % of MoS₂ secondary phase, more preferably about 0 mol % of MoS₂ secondary phase.

In the present description and in the following claims, the term "wet chemical route" is used to indicate that the sulfur-molybdenum cluster composition is obtained from a precursor deposited by a "wet chemistry" method (*i.e.* chemistry done in the liquid phase such as a spin coating or a dip coating method) which, in this specific case, involves the deposition of a fluid on a substrate. Conversely, the above-mentioned pulsed laser deposition technique (*i.e.* physical vapor deposition, gas phase deposition) is not a "wet chemistry" method as it involves the condensation of a vaporized form of a material with a high power pulsed laser beam focused on a substrate inside a vacuum chamber. Another commonly used thin-film deposition technique is the "sputtering" technique. "Sputtering" is a process comprising ejecting atoms from a solid target material due to bombardment of the target by energetic particles. Evidently, "sputtering" is not a "wet chemistry" method.

In the present description and in the following claims, the term "calcinating" is used to indicate the decomposition or reaction of oxidation of a material due to heat in the presence of oxygen.

In the present description and in the following claims, the term "sulfurizing" is used to indicate the heat treatment of a material with sulfur or a sulfur compound such as hydrogen sulfide (H₂S), optionally in presence of a carrier gas such as Ar or H₂, preferably, H₂.

In the present description and in the following claims, the term "reducing" is used to indicate the heat treatment of a material with a reducing agent, for example hydrogen (H₂).

In the present description and in the following claims, the term "chalcogenide" is used to indicate a compound containing a chalcogen.

In the present description and in the following claims, the term "about" is intended to indicate values independently deviating within 10 % margin, and for example within 5 % margin from the cited values. In the present description and in the following claims, a composition around the stoichiometric composition (*e.g.* Cu₂Mo₆S₈) indicates a composition in which each element may independently deviate from the respective stoichiometric value up to 10 % mol, and for example up to 5 % mol (*e.g.* Cu_{1.9}Mo_{6.3}S_{7.6}).

In the present description and in the following claims x and y are real numbers.

The process may comprise adding any amount, even a small amount, for example from about 0.2 mg to about 20 mg, preferably, from about 0.5 mg to about 5 mg, of a second molybdenum source (e.g. molybdenum containing compound) such as a molybdenum sulfide source, for example as a powder or a film such as a powder or a film of M'_{y}Mo₆S₈ or MoS₂ (commercially available) in a crucible where the film, which is prepared by calcinating and sulfurizating the precursor, is placed for the reduction treatment. While the prior art reduction methods are in fact irreproducible and particularly difficult to be performed due to the very small amount of matter being reduced, the addition of a second molybdenum source enables a better self-controlled reduction of the sulfur-molybdenum cluster composition because of the *in situ* competing reduction of the second molybdenum source. In fact, the second molybdenum source may comprise molybdenum sulfides. Preferably, a molybdenum sulfide used as a second molybdenum source has a melting point higher than the reduction temperature and is H₂ reducible in the temperature range of the reduction temperature. Preferably, a powder of M'_{y}Mo₆S₈ or MoS₂ is be placed on the crucible between the film and an inlet configured to provide the reducing agent, as shown for example in Figure 6 depicting a scheme of the crucible (MoS₂ or CuₓMo₆S₈ powder + thin film) in a furnace during the reduction (around 2 mg of powder). Preferably, the powder is used several times.

In some embodiments, the second molybdenum source is placed in a position adjacent to the position in which the precursor is placed, preferably upstream of the precursor with respect to the direction of the flow of the reducing agent (Figure 6). In some embodiments, the second molybdenum source does not contact the precursor. For example, the second molybdenum source may be placed at a distance ranging from about 0.1 cm to about 20 cm away from the precursor, preferably from about 0.5 cm to about 5 cm, for example at about 1 cm.

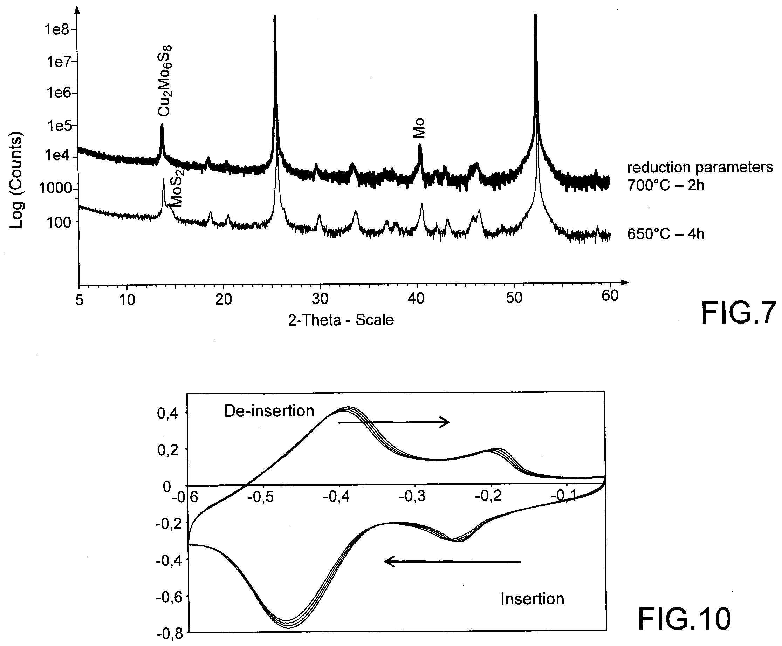

For example, the addition of 2 mg of Cu₂Mo₆S₈ or MoS₂ powder leads to reproducibly obtaining Cu₂Mo₆S₈ thin films free of MoS₂ secondary phase. Moreover, different thicknesses of Cu₂Mo₆S₈ thin films (for example, from 20 nm to 2 µm) can be obtained with very small amount of secondary phases and particularly substantially free of MoS₂ secondary phase. In addition, the use of the powder in combination with an increase of 50°C for the reduction temperature (700°C instead of 650°C) leads to the complete elimination of MoS₂. In that case, only a low amount of Mo secondary phase can be observed. The duration of the reduction can also be reduced by 2h (Figure 7). Note that the temperature increase without the addition of the powder does not lead to the reproducible formation of the phase. Indeed, it merely leads to an excessive reduction of the thin films with the presence of large amounts of Mo and Cu secondary phases.

While the present disclosure describes a specific method for the synthesis of a Cu₂Mo₆S₈ material such as a thin film which allows the reproducible formation of the phase with no MoS₂. The present disclosure can be extended to other cations: Ni for example in MₓMo₆S₈. The thin films can be obtained on insulating or conductive substrates and can be used for chemical or electrochemical insertion/de-insertion reactions.

In some embodiments, the sulfur-molybdenum cluster composition has the following formula: MₓMo₆S₈ wherein M is at least one metal other than molybdenum and 0 ≤ x < 4. In some embodiments, 1 ≤ x ≤ 3. In some embodiments, the sulfur-molybdenum cluster composition has the following formula: M₂Mo₆S₈. Depending on the size and on the valency of the cation, x ranges more preferably from about 1 to about 2 in some embodiments, and from about 2 to about 3 in other embodiments. For example, x ranges preferably from about 2 to about 3 for CuₓMo₆S₈ based materials such as thin films and electrode sensors, x may as well be 0 for Mo₆S₈ based materials such as thin films and electrode sensors.

In some embodiments, M is at least one cationic alkali metal or transition metal or mixture thereof. Preferably, M is selected from the group comprising Cu, Ni, Co, Zn, Pb, Mn, Cd, Li, and Mg, more preferably, M is Cu. In some embodiments, the sulfur-molybdenum cluster composition has a general formula of Cu₂Mo₆S₈.

In some embodiments, the second molybdenum source is a sulfur-molybdenum compound, salt thereof, or mixture thereof. For example, the second molybdenum source is a molybdenum sulfide source. Preferably the second molybdenum source is MoS₂, or M'_{y}Mo₆S₈ wherein M' is at least one metal other than molybdenum and 0 ≤ y < 4, preferably, 1 ≤ y ≤ 3, more preferably, y is equal to about 2.

In some embodiments, M' is a cationic alkali metal or transition metal compound, salt thereof, or mixture thereof. Preferably, M' is selected from the group comprising Cu, Ni, Co, Zn, Pb, Mn, Cd, Li, and Mg, more preferably, M' is Cu. In some embodiments, the second molybdenum source is M'₂Mo₆S₈, for example Cu₂Mo₆S₈.

In some embodiments, the precursor is a fluid deposited on a substrate. Preferably, the precursor is a fluid deposited by spin coating or dip coating. For example, the precursor is a fluid deposited at least once or successively on a substrate thereby forming a material having a thickness less than 5 µm, for example ranging from about 20 nm to about 5 µm, preferably from about 50 nm to about 3 µm, more preferably, from about 100 nm to about 2 µm. In some embodiments, the precursor is deposited from 1 to 20 times, preferably, from 3 to 10 times, for example, 5 times. In some embodiments, the substrate has a non planar surface. In some embodiments the substrate is deposited on at least one face of the substrate. In some embodiments the precursor is dried (typically at 100°C for a few minutes such as from about 1 minute to about 10 minutes) and/or calcinated between each deposition.

In some embodiments, the sulfur-molybdenum cluster composition is in the form of a thin film having a thickness ranging from about 20 nm to about 5 µm. In some embodiments, the precursor is deposited on at least one surface of the substrate, preferably on at least 2 opposite surfaces. In some embodiments, the at least one surface is non planar. In some embodiments, the at least one surface is greater than 0.5 cm², for example greater than 1.5 cm², such as greater than 2 cm² or 6.45 cm² (1 square inch).

In some embodiments, the precursor comprises a compound comprising at least one metal other than molybdenum such as a cationic alkali metal or transition metal compound, salt thereof, or mixture thereof. Preferably, the at least one metal other than molybdenum is selected from the group comprising Cu, Ni, Co, Zn, Pb, Mn, Cd, Li, and Mg. Preferably, the at least one metal other than molybdenum is a transition metal nitrate complex, a transition metal acetate complex, or a mixture thereof.

In some embodiments, the first molybdenum source (e.g. molybdenum containing compound) is a hexavalent molybdenum compound, salt thereof, or mixture thereof. Preferably, the first molybdenum source is a heptamolybdate salt, preferably, an ammonium heptamolybdate, more preferably, (NH₄)₆Mo₇O₂₄ 4H₂O.

In some embodiments, the sulfurization is carried under the flow of a mixture of hydrogen sulfide (H₂S) and a carrier gas, for example Argon or hydrogen (H₂), preferably H₂. For example, the mixture comprises from about 1 mol % to about 30 mol % of H₂S in H₂, preferably, from about 5 mol % to about 20 mol %, more preferably, about 10 mol %. In some embodiments, the reduction is carried under hydrogen (H₂) flow in the range from about 5 to about 30 cm³/min, preferably from about 15 to about 20 cm³/min.

In some embodiments, the precursor is a fluid comprising an alcohol and a carboxylic acid, which together optionally polymerize into a polyester resin having a viscosity ranging from about 0.01 Pa.s (10 cP) to about 0.1 Pa.s (100 cP), preferably, from about 0.02 Pa.s (20 cP) to about 0.06 Pa.s (60 cP). In some embodiments, the precursor becomes a polymeric resin in which the metal ions are trapped and dispersed at the molecular level in an organic network. The polyesterification reaction may be carried out in solvents, and for example in aqueous liquids.

In some embodiments, the alcohol is a polyalcohol and is for example ethylene glycol, sorbitol, glycerol, or mixture thereof and the carboxylic acid is a polycarboxylic acid and is for example citric acid, malonic acid, succinic acid, glutaric acid, malic acid, or mixture thereof. In some embodiments, the polyesterification reactions are carried out for example at a temperature ranging from about 40°C to about 150°C, for example from about 60°C to about 120°C, for example at about 80°C, and optionally under solution stirring. In some embodiments, the polyesterification reactions are performed for at least 5 minutes, for example for about 20 minutes depending on the temperature of the system.

In some embodiments, an amount (A) of the first molybdenum source is solubilized in a first solution containing a first amount (CA1) of carboxylic acid, preferably, an aqueous carboxylic acid solution, such as a water and citric acid mixture. The solubilization of the first molybdenum source is usually done at room temperature but may be carried at a temperature ranging from about 10°C to about 110°C. In some embodiments, the solubilization of the first molybdenum source is usually performed under a minute, but depending on the molybdenum source, the solubilization may be performed over a minute, for example for at least 5 min. In some embodiments, [1:5] ≤ [A:CA1] ≤ [1:1] (*i.e.* the molar ratio of A over CA1 ranges from about 1/5 to about 1/1), preferably, [1:4] ≤ [A:CA1] ≤ [1:2], more preferably, [A:CA1] = [1:3].

In some embodiments, an amount (B) of the compound comprising at least one metal other than molybdenum is solubilized in a second solution containing a second amount (CA2) of carboxylic acid, preferably, an aqueous carboxylic acid solution, such as a water and citric acid mixture. The solubilization of the compound comprising at least one metal other than molybdenum may be carried for example at a temperature ranging from about 10°C to about 110°C, for example from about 60°C to about 90°C, for example at about 80°C. In some embodiments, the solubilization of the compound comprising at least one metal other than molybdenum is performed under a minute, but depending on said source, the solubilization may be performed over a minute, for example for at least 5 min. In some embodiments, [1:5] ≤ [B:CA2] ≤ [1:1] (*i.e.* the molar ratio of B over CA2 ranges from about 1/5 to about 1/1), preferably, [1:4] ≤ [B:CA2] ≤ [1:2], more preferably, [B:CA2] = [1:3].

In some embodiments, [1:10] ≤ [B:A] ≤ [1:1] (*i.e.* the molar ratio of B over A ranges from 1/10 to about 1/1), preferably, [1:4] ≤ [B:A] ≤ [1:2], more preferably, [B:A] = [1:3].

In some embodiments, the weight ratio of the total amount of carboxylic acids over the total amount of alcohols present in the precursor ranges from about 1/4 to about 3/2, preferably from about 1/3 to about 1/1, more preferably about 2/3.

In some embodiments, the excess of solvent is removed from the resin, for example by solvent evaporation. In some embodiments, the viscosity of the resin is increased in a gradual manner, for example by evaporation of a solvent such as water or alcohols. In some embodiments, the viscosity of the resin may be decreased in a gradual manner by addition of solvents.

In some embodiments, the calcination is performed at a temperature ranging from about 250°C to about 600°C, preferably, from about 300°C to about 550°C, more preferably, at about 400°C. Preferably, the calcination is performed from about 1 hour to about 5 hours, preferably, from about 2 hours to about 4 hours, more preferably, for about 3 hours.

In some embodiments, the sulfurization is performed at a temperature ranging from about 450°C to about 750°C, preferably, from about 500°C to about 700°C, more preferably, at about 600°C. Preferably, the sulfurization is performed from about 1 hour to about 3 hours, preferably, for about 2 hours.

In some embodiments, the reduction is performed at a temperature ranging from about 600°C to about 850°C, more preferably 650°C to about 800°C, more preferably at about 700°C. Preferably, the reduction is performed from about 1 hour to about 6 hours, preferably, from about 90 min to about 270 minutes, more preferably, for about 2 hours.

In some embodiments, the calcination, sulfurization, and/or reduction can be performed by gradually increasing the temperature until a final calcination, sulfurization, and/or reduction temperature, for example by increasing the temperature at a predetermined heating rate, for example from about 1°C to about 10°C per minute, preferably at about 5°C per minute.

As a result of the removal of the MoS₂ secondary phase, the present disclosure strongly improves the electrochemical behavior of the films, as shown in Figure 8 (curve of de-insertion by voltammetry after insertion of the Cd by chronoamperometry at -500 mV during 1 min for [Cd²⁺] = 10⁻¹ mol.L⁻¹ and during 5 min for [Cd²⁺] = 10⁻³ mol.L⁻¹). The harmful influence of the MoS₂ on the electrochemical behavior can be explained by the increase of the thin film resistivity with the increase of MoS₂ amount. For example, Table 1 shows the resistivity of a Cu₂Mo₆S₈ thin film decreasing from 3.3 10⁻² Ω.cm to 3.3 10⁻³ Ω.cm as the amount of MoS₂ secondary phase is decreased.

**Table 1: influence of MoS₂ secondary phase on the electrochemical behavior of Co₂Mo₆S₈.**

| DRX (intensity of the main peak of Cu₂Mo₆S₈)/(intensity of the main peak of MoS₂) | Resistivity (Ω.cm) |
|---|---|
| 2.5 | 3.3 10⁻² |
| 5.6 | 6.8 10⁻³ |
| no MoS₂ | 3.3 10⁻³ |

In some embodiments, the film of sulfur-molybdenum cluster composition is substantially free of MoS₂ secondary phase or has a low electrical resistivity suitable for electrochemistry experiment such as less than about 3.3 10⁻² Ω.cm, preferably less than about 6.8 10⁻³ Ω.cm, more preferably less than about 3.3 10⁻³ Ω.cm. Preferably, the film of sulfur-molybdenum cluster composition having an electrical resistivity less than about 3.3 10⁻² Ω.cm has the following formula: MₓMo₆S₈ wherein M is at least one metal other than molybdenum and 0 ≤ x < 4, preferably 1 ≤ x ≤ 3. Depending on the size and on the valency of the cation, x ranges more preferably from about 1 to about 2 in some embodiments, and from about 2 to about 3 in other embodiments. For example, x ranges preferably from about 2 to about 3 for CuₓMo₆S₈ based thin films and electrode sensors, x may as well be 0 for Mo₆S₈ based thin films and electrode sensors. In some embodiments, M is at least one cationic alkali metal or transition metal or mixture thereof. Preferably, M is selected from the group comprising Cu, Ni, Co, Zn, Pb, Mn, Cd, Li, and Mg, more preferably, M is Cu. In some embodiments the sulfur-molybdenum cluster composition having an electrical resistivity less than about 3.3 10-² Ω.cm has a general formula of Cu₂Mo₆S₈.

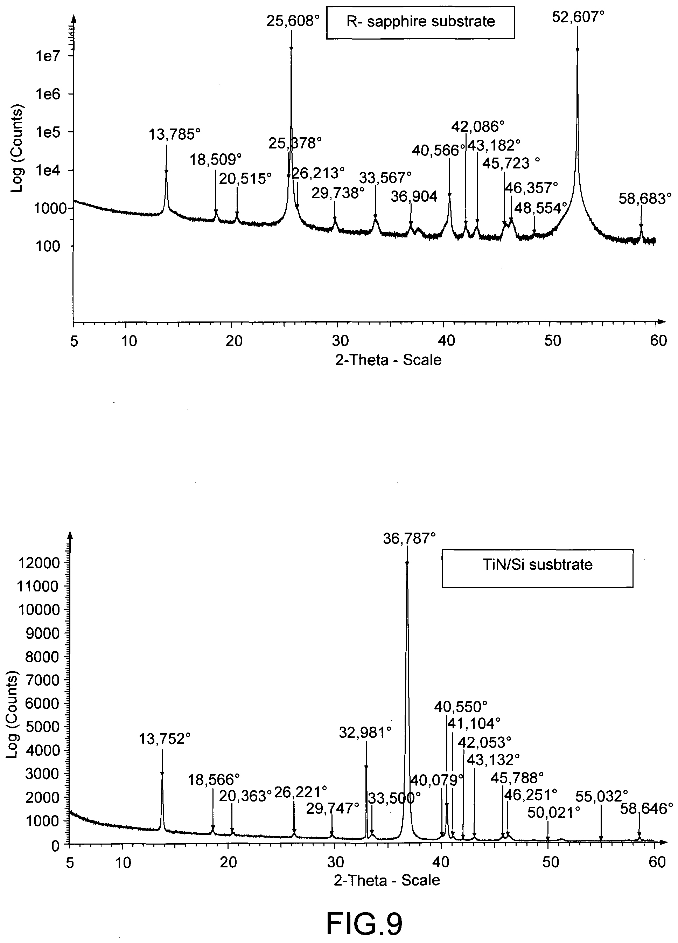

With this process, the thin films can be deposited on insulating substrates (sapphire for example) or conductive substrates (TiN/Si for example), as shown in Figures 9 and 10 (conductive substrates are required to realize electrodes for sensors for instance). For example, Figure 10 shows a curve of de-insertion by voltammetry after insertion of Cd by chronoamperometry at -500 mV during 1 min for [Cd²⁺] = 10⁻¹ mol.L⁻¹ (medium of CdSO₄ 10⁻¹ mol.L⁻¹ + Na₂SO₄ 10⁻¹ mol.L⁻¹). Other substrates, suitable to the treatments under H₂S/H₂ and H₂ gas flows, were successfully experimented such as Ti/Si, glassy carbon, Mo coated substrates and Mo sheet. Thus in some embodiments, the substrate can be either conductive or nonconductive (insulating). Preferably, the substrate is selected from the group comprising alumina, sapphire, TiN/Si, Ti/Si, Mo sheet, and glassy carbon.

Advantageously, embodiments of the present disclosure provide a new chemical route compatible with future development in industry and allowing to work not only in aqueous media, but also at lower temperature (e.g. compared to pulsed laser deposition or "sputtering" techniques), at lower cost (*e.g.* low cost precursors and simple deposition equipment), and at laboratory atmosphere (*e.g.* without a vacuum chamber). For example, spin coating gives access to large area samples (*e.g.* higher than 6.45 cm²) and is compatible with a microelectronic process for integration in devices; dip coating enables coating of non planar surfaces and/or two-sides samples and is adaptable to high volume orders requiring fast delivery.

Further, the "Directive Cadre sur l'Eau" (DCE) of October 23th 2000 has identified priority substances which amounts have to be strongly reduced in water. Cd, Hg, Pb, Ni, Zn are part of these substances. The DCE implementation in France, as well as its equivalent across Europe, in the US, and throughout the world is a major priority and therefore, there is a real need of methods for rapid and efficient detection.

The industries using processes based on insertion/de-insertion reactions may be of interested by the present disclosure. The relevant sectors are in the area of the electrochemical sensors for environment. The companies working already on the detection of pollutants using electrochemical methods may be particularly interested. The use of compositions of the present disclosure as potential components of sensors is a robust technique that is an improvement compared to extremely fragile microelectrodes.

Therefore, in some embodiments, the film of sulfur-molybdenum cluster composition of the present disclosure is used in an electrochemical apparatus. In some embodiments, the electrochemical apparatus is used in aqueous and/or non aqueous media. In some embodiments, the electrochemical apparatus is an electrochemical sensor for example for detecting and/or quantifying the presence of at least one metal cation in a sample. Preferably, the at least one metal cation is selected from the group comprising alkali metals, alkaline earth metals, transition metals, post-transition metals, and lanthanides. Preferably, the at least one metal cation is selected from the group comprising Cd, Zn, Cu, Ni, Pb, Mn, Cr, Hg, Sn, Ag, Co, Fe, Li, Na, Mg, Ho, and Sm. In some embodiments, the at least one metal cation is selected from the group comprising Cd, Hg, Pb, Ni, Zn, Cu, Mn, Co. In some embodiments, the at least one metal cation is Na and/or Mg.

Another sector that may be interested by the present disclosure is the sector of energy. Indeed, these sulfur-molybdenum materials can be used as electrodes for batteries, for example by insertion of the lithium or magnesium cations. These materials can also be incorporated as thin film layer(s) in chalcogenide type film based integrated devices that may for example be used as electrode. For example, in some embodiments, the thin film of sulfur-molybdenum cluster composition may be integrated in a thin-film photovoltaic cell as, for example, a buffer layer and/or a bottom electrode for photovoltaic applications.

The composition of the films was analyzed by energy-dispersive X-ray spectroscopy (EDS) using a Jeol JSM 6400 scanning electron microscope equipped with an ISIS Oxford analyzer.

XRD analyses were carried out with a BRUKER D8 ADVANCE diffractometer using a monchromatized CuKalphal radiation and equipped with a LYNXEYE detector Electrochemical analyses were recorded on a Radiometer PGZ 301 potentiostat / galvanostat driven by a computer (Voltamaster4 software) or on a BioLogic VSP potentiostat / galvanostat (EC-lab software) at room temperature.

## Claims

1. A process for the preparation of a film comprising a sulfur-molybdenum cluster composition, the process comprising:
preparing a precursor comprising a first molybdenum compound,
calcinating the precursor,
sulfurizing the precursor, and
reducing the precursor in presence of a second molybdenum compound and of a reducing agent.

2. The process according to claim 1, wherein the reducing a precursor comprises placing the second molybdenum compound in a position adjacent to the position in which the precursor is placed.

3. The process according to claim 1 or 2, wherein the reducing a precursor comprises placing the second molybdenum compound upstream of the precursor with respect to the direction of the flow of the reducing agent.

4. The process according to any preceding claim, wherein the second molybdenum compound is a molybdenum sulfide compound.

5. The process according to any preceding claim, wherein the second molybdenum compound is MoS₂, or M'_{y}Mo₆S₈ wherein M' is at least one metal other than molybdenum and 0 ≤ y < 4.

6. The process according to any preceding claim, wherein the preparing a precursor comprises depositing the precursor as a fluid on at least one surface of a substrate.

7. The process according to claim 6, wherein the substrate is conductive or nonconductive.

8. The process according to claim 6 or 7, wherein the substrate is selected from the group comprising alumina, sapphire, TiN/Si, Ti/Si, Mo sheet, and glassy carbon.

9. The process according to any of claims 6 to 8, wherein the depositing the precursor comprises depositing the precursor by spin coating or dip coating.

10. The process according to any preceding claim, wherein the precursor further comprises a compound comprising at least one metal other than molybdenum.

11. The process according to any preceding claim, wherein the sulfur-molybdenum cluster composition has the following formula: MₓMo₆S₈ wherein M is at least one metal other than molybdenum and 0 ≤ x < 4.

12. The process according to any preceding claim, wherein the sulfur-molybdenum cluster composition has the formula Cu₂Mo₆S₈.

13. The process according to any of claims 1 to 10, wherein the sulfur-molybdenum cluster composition has the formula Mo₆S₈.

14. The process according to any preceding claim, wherein the first molybdenum compound is a hexavalent molybdenum compound, salt thereof, or mixture thereof.

15. A film obtainable by the process according to any preceding claim.

16. An electrochemical apparatus comprising at least one film according to claim 15.

17. A method for detecting and/or quantifying the presence of at least one metal cation in a sample by insertion/de-insertion reaction of the at least one metal cation on at least one film according to claim 15.

18. An electrode comprising at least one film according to claim 15.

19. A chalcogenide containing film based integrated device comprising at least one film according to claim 15.

## Patentansprüche

1. Verfahren für die Herstellung eines Films, der eine Schwefel-Molybdän-Cluster-Zusammensetzung umfasst, wobei das Verfahren umfasst:
Herstellen eines Vorläufers, der eine erste Molybdänverbindung umfasst,
Calzinieren des Vorläufers
Schwefeln des Vorläufers, und
Reduzieren des Vorläufers in Anwesenheit einer zweiten Molybdänverbindung und eines Reduktionsmittels.

2. Verfahren nach Anspruch 1, wobei das Reduzieren eines Vorläufers das Anordnen der zweiten Molybdänverbindung an einer Stelle, die zu der Stelle, an der der Vorläufer angeordnet ist, benachbart ist, umfasst.

3. Verfahren nach Anspruch 1 oder 2, wobei das Reduzieren eines Vorläufers das Anordnen der zweiten Molybdänverbindung in Bezug auf die Strömungsrichtung des Reduktionsmittels vor dem Vorläufer umfasst.

4. Verfahren nach einem vorhergehenden Anspruch, wobei die zweite Molybdänverbindung eine Molybdänsulfidverbindung ist.

5. Verfahren nach einem vorhergehenden Anspruch, wobei die zweite Molybdänverbindung MoS₂ oder M'_{y}Mo₆S₈ ist, wobei M' wenigstens ein anderes Metall als Molybdän ist und wobei 0 ≤ y < 4 ist.

6. Verfahren nach einem vorhergehenden Anspruch, wobei das Herstellen eines Vorläufers das Ablagern des Vorläufers als ein Fluid auf wenigstens einer Oberfläche eines Substrats umfasst.

7. Verfahren nach Anspruch 6, wobei das Substrat leitend oder nichtleitend ist.

8. Verfahren nach Anspruch 6 oder 7, wobei das Substrat aus der Gruppe ausgewählt wird, die Aluminiumoxid, Saphir, TiN/Si, Ti/Si, eine Mo-Lage und Glaskohlenstoff umfasst.

9. Verfahren nach einem der Ansprüche 6 bis 8, wobei das Ablagern des Vorläufers das Ablagern des Vorläufers durch Schleuderbeschichtung oder Tauchbeschichtung umfasst.

10. Verfahren nach einem vorhergehenden Anspruch, wobei der Vorläufer ferner eine Verbindung umfasst, die wenigstens ein anderes Metall als Molybdän umfasst.

11. Verfahren nach einem vorhergehenden Anspruch, wobei die Schwefel-Molybdän-Cluster-Zusammensetzung die folgende Formel aufweist: MₓMo₆S₈, wobei M wenigstens ein anderes Metall als Molybdän ist und wobei 0 ≤ x < 4 ist.

12. Verfahren nach einem vorhergehenden Anspruch, wobei die Schwefel-Molybdän-Cluster-Zusammensetzung die Formel Cu₂Mo₆S₈ aufweist.

13. Verfahren nach einem der Ansprüche 1 bis 10, wobei die Schwefel-Molybdän-Cluster-Zusammensetzung die Formel Mo₆S₈ aufweist.

14. Verfahren nach einem vorhergehenden Anspruch, wobei die erste Molybdänverbindung eine sechswertige Molybdänverbindung, ein Salz davon oder ein Gemisch davon ist.

15. Film, der durch das Verfahren nach einem vorhergehenden Anspruch erhalten werden kann.

16. Elektrochemische Vorrichtung, die wenigstens einen Film nach Anspruch 15 umfasst.

17. Verfahren zum Detektieren und/oder Quantifizieren der Anwesenheit wenigstens eines Metallkations in einer Probe durch eine Einschiebungs-/ Ausschiebungsreaktion des wenigstens einen Metallkations auf wenigstens einem Film nach Anspruch 15.

18. Elektrode, die wenigstens einen Film nach Anspruch 15 umfasst.

19. Integrierte Vorrichtung auf der Grundlage eines Chalkogenid-haltigen Films, die wenigstens einen Film nach Anspruch 15 umfasst.

## Revendications

1. Procédé de préparation d'un film comprenant une composition d'agrégat de soufre-molybdène, le procédé comprenant :
une préparation d'un précurseur comprenant un premier composé molybdène,
une calcination du précurseur,
une sulfurisation du précurseur, et
une réduction du précurseur en présence d'un second composé molybdène et d'un agent réducteur.

2. Procédé selon la revendication 1, dans lequel la réduction d'un précurseur comprend un placement du second composé molybdène dans une position adjacente à la position dans laquelle le précurseur est placé.

3. Procédé selon la revendication 1 ou 2, dans lequel la réduction d'un précurseur comprend un placement du second composé molybdène en amont du précurseur par rapport à la direction de l'écoulement de l'agent réducteur.

4. Procédé selon l'une quelconque des revendications précédentes, dans lequel le second composé molybdène est un composé de sulfure de molybdène.

5. Procédé selon l'une quelconque des revendications précédentes, dans lequel le second composé molybdène est MoS₂ ou M'_{y}Mo₆S₈ où M' est au moins un métal différent du molybdène et 0 ≤ y < 4.

6. Procédé selon l'une quelconque des revendications précédentes, dans lequel la préparation d'un précurseur comprend le dépôt du précurseur sous forme de fluide sur au moins une surface d'un substrat.

7. Procédé selon la revendication 6, dans lequel le substrat est conducteur ou non conducteur.

8. Procédé selon la revendication 6 ou 7, dans le quel le substrat est choisi dans le groupe comprenant de l'alumine, du saphir, TiN/Si, Ti/Si, une feuille de Mo et du carbone vitreux.

9. Procédé selon l'une quelconque des revendications 6 à 8, dans lequel le dépôt du précurseur comprend le dépôt du précurseur par revêtement par centrifugation ou revêtement par immersion.

10. Procédé selon l'une quelconque des revendications précédentes, dans lequel le précurseur comprend en outre un composé comprenant au moins un métal différent du molybdène.

11. Procédé selon l'une quelconque des revendications précédentes, dans lequel la composition d'agrégat de soufre-molybdène a la formule suivante : MₓMo₆S₈ où M est au moins un métal différent du molybdène et 0 ≤ x < 4.

12. Procédé selon l'une quelconque des revendications précédentes, dans lequel la composition d'agrégat de soufre-molybdène a la formule Cu₂Mo₆S₈.

13. Procédé selon l'une quelconque des revendications 1 à 10, dans lequel la composition d'agrégat soufre-molybdène a la formule Mo₆S₈.

14. Procédé selon l'une quelconque des revendications précédentes, dans lequel le premier composé molybdène est un composé molybdène hexavalent, un de ses sels ou un de ses mélanges.

15. Film obtenu par le procédé selon l'une quelconque des revendications précédentes.

16. Appareil électrochimique comprenant au moins un film selon la revendication 15.

17. Procédé de détection et/ou de quantification de la présence d'au moins un cation métallique dans un échantillon par réaction d'insertion/dé-insertion de l'au moins un cation métallique sur au moins un film selon la revendication 15.

18. Électrode comprenant au moins un film selon la revendication 15.

19. Dispositif intégré à base de film contenant du chalcogénure comprenant au moins un film selon la revendication 15.
